# EUROPÄISCHE PATENTSCHRIFT

(11) **EP 0 460 344 B1**
(45) Veröffentlichungstag und Bekanntmachung des Hinweises auf die Patenterteilung: **12.04.1995**
(21) Anmeldenummer: 91100026.3
(22) Anmeldetag: 02.01.1991
(51) Int. Cl.: B08B 3/06, A61L 2/26

(54) **Einrichtung zum Reinigen und Sterilisieren von Gegenständen, insbesondere pharmazeutischen Verschlusselementen**
Device for cleaning and sterilizing of parts, especially pharmaceutical fasteners
Dispositif pour le nettoyage et la stérilisation particulièrement des fermetures d'éléments pharmaceutiques

(30) Priorität: 05.06.1990 DE 4018023
(43) Veröffentlichungstag der Anmeldung: 11.12.1991
(73) Patentinhaber: Völpel, Dietwart, D-85356 Freising (DE)
(72) Erfinder: Völpel, Dietwart, D-85356 Freising (DE)
(74) Vertreter: von Bezold, Dieter, Dr.

(56) Entgegenhaltungen:
- DE-C- 549 217
- DE-C- 3 248 555
- US-A- 3 487 840
- US-A- 3 853 622

## Beschreibung

Die vorliegende Erfindung geht aus von einer Einrichtung zum Reinigen und Sterilisieren von Gegenständen, insbesondere Kleinteilen, wie pharmazeutischen Verschlußelementen, mit
- einer Autoklavenkammer, welche eine erste Stirnseite mit einer durch eine erste Verschlußvorrichtung dicht verschließbaren Beschickungsöffnung, eine dieser entgegengesetze zweite Stirnseite mit einer durch eine zweite Verschlußvorrichtung dicht verschließbaren Entladeöffnung und eine Umfangswand aufweist,
- einer sich in der Autoklavenkammer befindenden Reinigungsvorrichtung, welche eine zur Aufnahme der zu reinigenden Gegenstände dienende Trommel mit durchbrochenem Mantel sowie eine Welle zur drehbaren Lagerung der Trommel aufweist,
- einer zum Antrieb der Trommel dienenden, außerhalb der Autoklavenkammer angeordneten Antriebsvorrichtung, die durch eine in einer Seite der Autoklavenkammer vorgesehene Durchbrechung mit der Trommel koppelbar ist, und
- einer Vorrichtung zum Einspeisen mindestens eines Fluids, wie einer Reinigungsflüssigkeit, in die Trommel.

An die Reinheit, Partikelfreiheit und Sterilität gewisser Gegenstände, insbesondere pharmazeutischer Verschlußelemente und anderer für den pharmazeutischen oder medizinischen Gebrauch bestimmter Artikel, werden sehr hohe Anforderungen gestellt. Andererseits soll die Reinigung und Sterilisierung zuverlässig und wirtschaftlich durchgeführt werden können. Aus der DE-C-32 48 555 ist eine Maschine zum Reinigen empfindlicher Kleinteile, wie pharmazeutischer Verschlußelemente bekannt, die eine im wesentlichen geschlossene Außentrommel enthält, die mit Zu- und Ableitungen für Behandlungsfluide verbunden ist. In der Außentrommel ist eine Innentrommel mit einer Hohlwelle, die mit einem Antriebsaggregat verbunden ist, drehbar gelagert. Die Hohlwelle ist mit einer Vorrichtung zum Einspeisen von Reinigungsflüssigkeit verbunden und hat Spritzdüsen, die in das Innere der Innentrommel gerichtet sind. Mit der Hohlwelle ist außerdem ein U-förmiges Spritzrohr verbunden, welches die Innentrommel in einer durch deren Drehachse gehenden Ebene U-förmig umgibt und Spritzdüsen aufweist, die zur Innenwand der Außentrommel hin gerichtet sind. Die Außentrommel weist eine dicht verschließbare Beschickungsöffnung auf, welche von einem unreinen Raum aus zugänglich ist, und eine dicht verschließbare Entladeöffnung, die von einem Sterilraum aus zugänglich ist.

Es sind auch Autoklaven bekannt, in die eine auf Rädern fahrbar gelagerte Reinigungsvorrichtung, die eine drehbare Trommel enthält, in Achsrichtung der Trommel von einem unreinen Raum aus einschiebbar und nach Reinigung und Sterilisierung der in der Trommel enthaltenen Gegenstände von einem Sterilraum aus entnehmbar ist.

Die oben erwähnten bekannten Einrichtungen lassen in verschiedener Hinsicht noch zu wünschen übrig. Bei der erstgenannten Maschine kann man die Reinigung nur in der fest eingebauten Trommel vornehmen, also die praktisch einen Autoklaven darstellende Außentrommel nicht für die Reinigung von Gegenständen, die in Wannen oder Trögen untergebracht sind, verwenden. Außerdem kann man mit der Füllung der Trommel erst nach deren Entladung beginnen und nicht die entleerte Trommel gegen eine vorbereitete, vorher gefüllte Trommel austauschen. Bei den Einrichtungen, die eine Autoklavenkammer und eine in diese einfahrbare Reinigungsvorrichtung enthalten, ist eine ausreichende Reinheit und Partikelfreiheit im allgemeinen nicht gewährleistet.

Der vorliegenden Erfindung liegt dementsprechend die Aufgabe zugrunde, eine Einrichtung zum Reinigen und Sterilisieren von Gegenständen, insbesondere pharmazeutischen Verschlußelementen, anzugeben, die höchsten Ansprüchen hinsichtlich Reinheit, Partikelfreiheit und Sterilität genügt und ein problemloses sowie wirtschaftliches Arbeiten gewährleistet.

Diese Aufgabe wird bei einer Einrichtung der eingangs angegebenen Art dadurch gelöst, daß die Antriebsvorrichtung mindestens eine von der als Hohlwelle ausgebildeten Trommelwelle getrennte Antriebs-Hohlwelle aufweist, die mit einem Ende dicht und drehbar durch die Seite der Autoklavenkammer in diese eingeführt ist und außerhalb der Autoklavenkammer mit einem Antriebsaggregat gekoppelt ist, und daß wenn die Reinigungs vorrichtung in der Autoklavenkammer angeordnet ist, die Antriebswelle und die Trommelwelle axial fluchten und mit einander gegenüberliegenden, komplementären Kupplungsteilen zur mechanischen Kopplung und zur Verbindung von in den Wellen verlaufenden Fluidkanälen miteinander koppelbar sind und voneinander entkoppelbar sind.

Weiterbildungen und vorteilhafte Ausgestaltungen der erfindungsgemäßen Einrichtung sind Gegenstand von Unteransprüchen.

Die vorliegende Einrichtung gewährleistet aufgrund ihrer Konstruktion eine optimale Reinigung und weitestgehende Partikelfreiheit sowie eine einwandfreie Sterilisation, die nach Wahl mit verschiedenen Verfahren durchgeführt werden kann. Während eine Trommelcharge gereinigt und sterilisiert wird, kann eine zweite Trommel vorbereitet und gefüllt werden, so daß die erste Trommel nach Entleerung gegen die gefüllte zweite Trommel ausgetauscht werden kann und sofort ein neuer Reinigungs- und Sterilisierungszyklus begonnen werden kann. Die Autoklavenkammer kann außerdem auch zur Reinigung von Gegenständen verwendet werden, die in Wannen, Trögen und dergleichen untergebracht sind oder auch von größeren Gegenständen, die z.B. auf einer geeigneten Palette oder einem mit Rollen versehenen Untersatz in die Autoklavenkammer eingeführt werden können. Dadurch, daß die Hohlwelle der Trommel und die Antriebswelle axial fluchtend durch eine einfache, abgedichtete Kupplungsvorrichtung koppelbar sind, wird die Gefahr, daß durch die Kupplungsvorrichtung unerwünschte Fremdkörper in die Autoklavenkammer eingeführt werden, weitestgehend verringert. Auch wenn die Autoklavenkammer ohne angekuppelte Reinigungstrommel verwendet wird, stellt der verbliebene Teil der Kupplungsvorrichtung keine Verschmutzungsgefahr dar. Außerdem kann das innere der Autoklavenkammer schnell und wirksam durch die nach außen gerichteten Düsen des Düsenrohres 44 gereinigt werden. Da die Autoklavenkammer praktisch keine Einbauten aufweist, läßt sie sich vielseitig verwenden

Besonders vorteilhaft ist es, wenn der Rahmen, auf dem die Reinigungstrommel zum Einführen in die Autoklavenkammer sowie zum Entnehmen aus dieser (beides durch die sich auf der unsterilen Seite befindliche Beschickungsöffnung) gelagert ist, von der in die Autoklavenkammer eingesetzten Trommel abgekoppelt und unabhängig von der Trommel aus der Autoklavenkammer entfernt werden kann, so daß der Rahmen sich während des Reinigungsprozesses nicht in der Autoklavenkammer befindet und daher auch keine Quelle für Verunreinigungen bilden kann.

Im folgenden werden Ausführungsbeispiele der Erfindung unter Bezugnahme auf die Zeichnungen näher erläutert. Es zeigen:
- Figur 1: eine schematische, teilweise geschnitte Seitenansicht einer Einrichtung gemäß einer Ausführungsform der Erfindung;
- Figur 2: einen schematischen Querschnitt durch eine Autoklavenkammer der Einrichtung gemäß Figur 1 und eine in diese eingefahrene Reinigungsvorrichtung,
- Figur 3: eine schematische Schnittansicht der Autoklavenkammer gemäß Figur 2 in einer die Achse einer Reinigungstrommel enthaltenden Ebene;
- Figur 4: einen etwas genaueren, vergrößerten Axialschnitt eines Teiles der Einrichtung gemäß Figur 1 bis 3;
- Figur 5: eine schematische Ansicht einer Kupplungsvorrichtung für die Einrichtung gemäß Figur 1 bis 4,
- Figur 6: eine Figur 1 entsprechende Darstellung einer weiteren, bevorzugten Ausführungsform der Erfindung und
- Figur 7: einen etwas genaueren, vergrößerten Axialschnitt von Teilen der Einrichtung gemäß Figur 6.

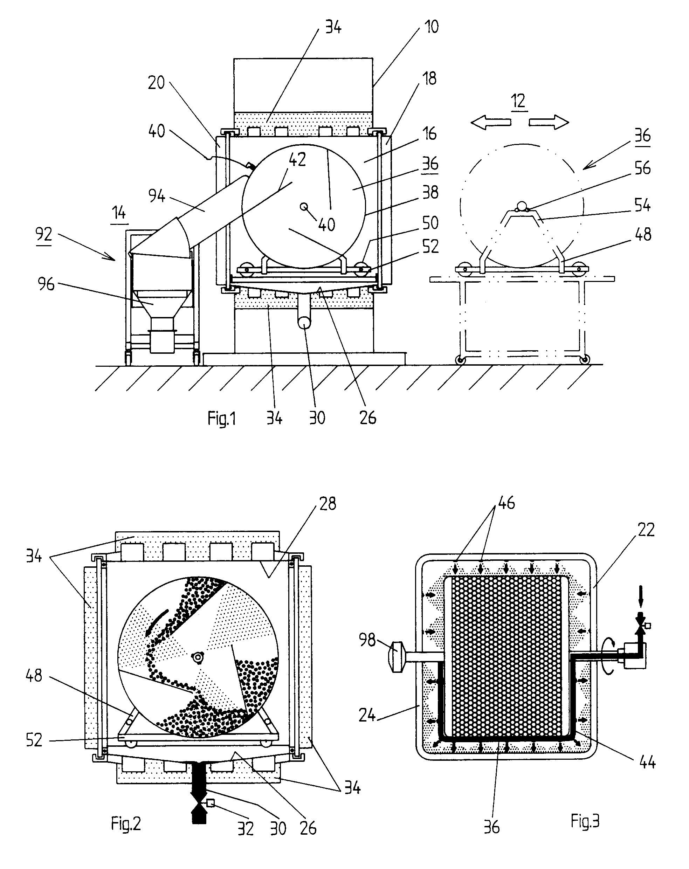

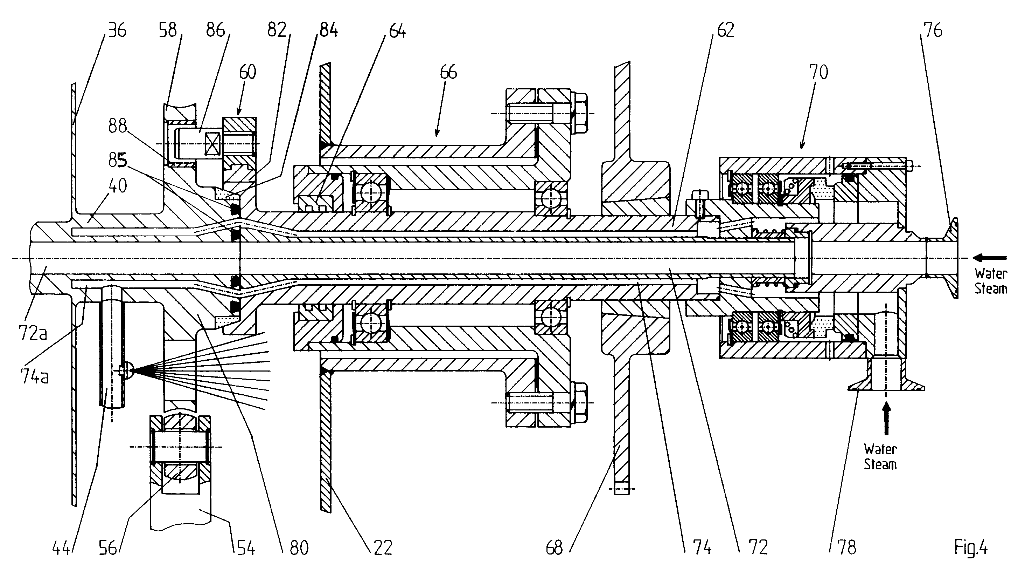

In Figur 1 bis 5 ist ein erstes Ausführungsbeispiel einer erfindungsgemäßen Einrichtung dargestellt, die zum Reinigen und Sterilisieren von pharmazeutischen Verschlußelementen, wie Gummistopfen bestimmt ist. Sie kann jedoch selbstverständlich auch für andere Fluidbehandlungen, wie Silikonisieren, und für die Behandlung anderer Gegenstände, an die entsprechende Reinheitsanforderungen gestellt werden, verwendet werden. Die dargestelle Einrichtung enthält ein Gehäuse 10, welches einen unreinen Raum 12 von einem sterilen Raum 14 trennt. Im Gehäuse 10 ist eine Autoklavenkammer 16 angeordnet, welche an einer ersten Stirnseite eine Beschickungsöffnung aufweist, welche durch eine vereinfacht als Türe 18 dargestellte Verschlußvorrichtung dicht verschließbar ist und auf der gegenüberliegenden Stirnseite eine Entladeöffnung, die durch eine als Türe 20 dargestellte Verschlußvorrichtung dicht verschließbar ist. Die Autoklavenkammer hat ferner zwei Seitenwände 22, 24 (Figur 3), einen Boden 26 und eine obere Wand 28. Der Boden 26 (Figur 1 und 2) ist trichterförmig und hat der tiefsten Stelle ein Abflußrohr 30, welches durch ein Ventil 32, insbesondere ein Magnetventil, verschließbar ist. Bei der dargestellten Ausführungsform sind Boden, Decke und die Türen mit einer nur schematisch angedeuteten Heizvorrichtung und/oder Wärmeisolierung 34 versehen.

In die Autoklavenkammer 16 ist von der Seite des unreinen Raumes 12 aus eine Reinigungsvorrichtung 36 einfahrbar. Die Reinigungsvorrichtung enthält bei dem vorliegenden Ausführungsbeispiel eine Reinigungstrommel 38 mit durchbrochenem Mantel, der eine Türe 40 zur Beschickung und Entleerung der Trommel aufweist. Die Trommel 38 sitzt auf einer Hohlwelle 40, welche als Spritzrohr dient und nach außen gerichtete Düsen aufweist, welche fächerförmige Fluidstrahlen liefern, die in Figur 2 durch gepunktete Sektoren dargestellt sind. In der Trommel sind drei Mitnehmerwände 42 angeordnet, die in einem Winkel von etwa 20° bezüglich der Radialrichtung geneigt sind. Die Trommel ist im wesentlichen so ausgebildet, wie es in der oben erwähnten DE-A-32 48 555 beschrieben ist, so daß sich eine weitere Erläuterung erübrigt.

Wie Figur 3 zeigt, ist mit der Hohlwelle ein U-förmiges Düsenrohr 44 verbunden, das in einer durch die Achse der Trommel gehenden Ebene verläuft und nach innen gerichtete Spritzdüsen aufweist, durch die die Außenseite der Trommel 38 oder andere Gegenstände, die sich im Inneren der Autoklavenkammer befinden, gereinigt werden können. An der Innenwand der Autoklavenkammer sind Sprühdüsen 46 (Figur 3) vorgesehen, die nach innen in Richtung auf die Trommel 36 hin gerichtet und mit einer geeigneten Fluidzuführung (nicht dargestellt) verbunden sind.

Die Reinigungsvorrichtung 36 hat einen Rahmen 48, der ein auf vier Rollen 50 fahrbar gelagertes Untergestell 52 und zwei von diesem hochstehende, etwa trapezförmige Bügel 54 aufweist, an deren oberem Ende die Hohlwelle 40 um eine Achse, die quer zur Fahrrichtung der Rollen 50 verläuft, drehbar gelagert ist. Die Bügel sind hierfür oben jeweils mit zwei nebeneinanderliegenden Lagerrollen 56 versehen. Wie Figur 4 für die eine Seite der Trommel zeigt, haben die Lagerrollen 56 eine etwas ballige Stirnfläche, auf der eine mit der Hohlwelle verbundene Lagerscheibe 58 läuft, die eine entsprechend konkave Stirnfläche hat. Die Lagervorrichtung auf der anderen Seite der Trommel ist entsprechend ausgebildet. Am Boden der Autoklavenkammer sind zweckmäßigerweise Rillen oder Schienen zur Führung der Rollen vorgesehen, um eine genaue Positionierung der Reinigungsvorrichtung in der Autoklavenkammer in Achsrichtung der Trommel zu gewährleisten.

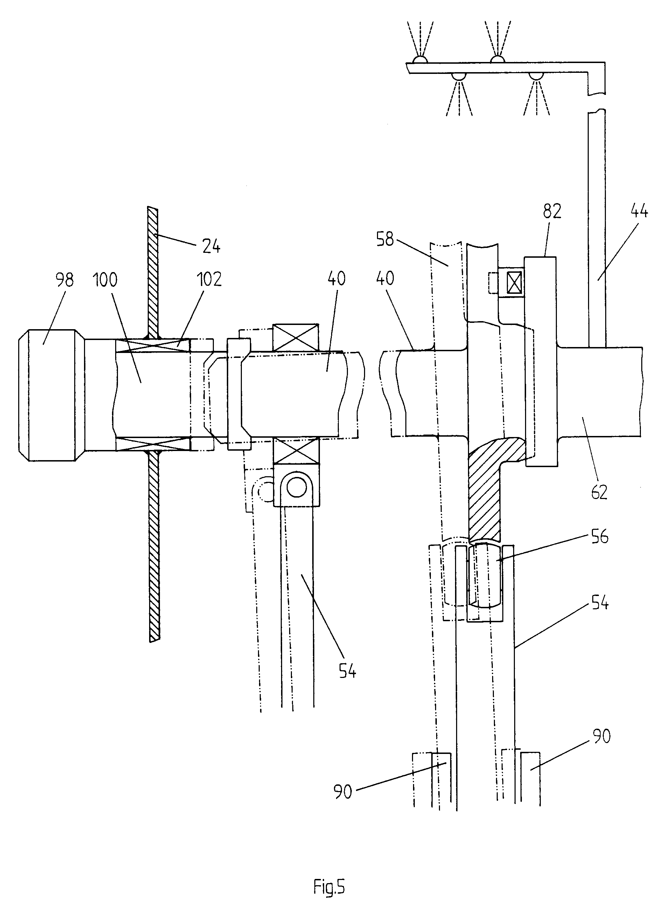

Die Hohlwelle ist über eine ausrückbare Kupplungsvorrichtung, die anhand von Figur 4 und 5 noch näher erläutert werden wird, mit einer hohlen Antriebswelle 62 sowohl mechanisch als auch hinsichtlich der in den Hohlwellen verlaufenden Fluidkanäle koppelbar. Die Antriebswelle 62 ist bezüglich der Seitenwand 22 z. B. durch eine labyrinthartig ausgebildete Dichtung 64 druck- und vakuumfest abgedichtet und außerhalb der Dichtung 64 durch Wälzlager 66 drehbar gelagert. Sie wird über ein Zahnrad 68 von einem nicht dargestellten drehzahlveränderlichen und in der Drehrichtung umsteuerbaren Antriebsvorrichtung angetrieben, das ein Getriebe und einen Elektromotor enthalten kann.

Am äußeren Ende der Antriebswelle ist ein Drehkopf 70 bekannter Bauart angeordnet. Bei der dargestellten Ausführungsform bildet die Antriebswelle einen zentralen Fluidkanal 72 und mindestens einen außeraxialen Fluidkanal 74, die mit getrennten Fluidanschlüssen 76 bzw. 78 des Drehkopfes in Verbindung stehen und jeweils die Einführung von Behandlungsfluiden, wie Wasser, Dampf, Reinigungslauge, Silikonisierungsflüssigkeit, Gas zur Sterilisierung, wie Ethylenoxid u.a.m. ermöglichen.

Die Kupplungsvorrichtung 60 enthält einen mit der Hohlwelle 40 der Reinigungstrommel verbundenen kegelstumpfförmigen Teil 80 mit relativ geringer Konizität. Die Antriebswelle 62 hat am inneren Ende einen Kupplungsteil 82, der eine zum kegelstumpfförmigen Teil 80 komplementäre Vertiefung aufweist. Die Fluidkanäle 72 und 74 münden in der Vertiefung des Kupplungsteiles 82 und der konisch zulaufende Teil hat entsprechende Kanäle 72a, 74a, die die Fortsetzung der Kanäle 72 bzw. 74 bilden, wobei der Kanal 72a die Düsen der Trommelwelle 40 und der Kanal 74a die Düsen des Düsenrohres 44 speist. Zur Abdichtung ist der Teil 80 mit einer äußeren ringförmigen Dichtung 84 mit konisch zulaufender Außenfläche versehen und außerdem mit zwei O-Ring-Dichtungen 85, die außerhalb und innerhalb der Mündung des außeraxial verlaufenden Fluidkanals 74 angeordnet sind.

Die Kupplungsvorrichtung 60 enthält ferner einen Mitnehmerstift 86, der in einem radialen Vorsprung des Kupplungsteiles 82 befestigt ist und in ein Loch 88 in der Lagerscheibe 58 eingreift, wenn die Kupplungsvorrichtung eingerückt ist.

Das Ein- und Ausrücken der Kupplungsvorrichtung 60 kann auf verschiedene Weise bewirkt werden. Wie Figur 5 zeigt, sind bei der dargestellten Ausführungsform die Bügel 54 oder ein Teil dieser Bügel in Achsrichtung der Trommel um einen kleinen Winkel schwenkbar, so daß die Hohlwelle 40 mit der Trommel von dem axial fixierten Kupplungsteil 82 so weit entfernt werden kann, daß der konisch zulaufende Teil 80 sowie der Mitnehmerstift 86 das Ein- und Ausfahren der Reinigungsvorrichtung nicht behindern. Der Schwenkbereich der Bügel 54 wird durch einen Anschlag 90 begrenzt, der in nicht dargestellter Weise am Rahmen befestigt ist. Die Schwenkung zwischen der ausgerückten Stellung (in Figur 5 gestrichelt dargestellt) und der eingekuppelten Stellung (in Figur 5 ausgezogen dargestellt) erfolgt bei der Ausführungsform gemäß Fig. 5 durch einen Pneumatikzylinder 98, der eine Welle 100 axial zu verschieben gestattet, die durch eine abgedichtete Lageranordnung drehbar, axial verschiebbar und dicht in der Seitenwand 22 der Autoklavenkammer 16 gelagert ist. Die Welle fluchtet mindestens im eingeschobenen Zustand axial mit der Trommelwelle 40 und übt auf diese eine so hohe Axialkraft auf, daß die Dichtigkeit der Kupplungsanordnung bei den verwendeten Fluiddrücken gewährleistet ist. Die Anordnung der Schwenkachsen der Bügel, die Länge der schwenkbaren Teile der Bügel und der Schwenkbereich sind vorteilhafterweise so bemessen, daß die Lagerscheibe 58 von den Rollen abgehoben wird, wenn die konischen Kupplungsteile zusammen gedrückt werden. Im Betrieb kann dann kein Abrieb an den Rollen und der Lagerscheibe auftreten. Entsprechendes gilt für eine auf der anderen Seite der Trommel befindliche Lagerscheibe, wenn die Trommel dort ebenso wie in Fig. 4 dargestellt gelagert ist. Alternativ kann die Trommelwelle auch an einem oder beiden Enden durch Gleitlager gelagert sein, wie dies in Fig. 5 für das linke Ende der Trommelwelle 40 dargestellt ist. Die Welle 100 kann eine Hohlwelle sein und wie die Antriebswelle 62 mit einer-Antriebsvorrichtung gekoppelt und/oder mit einem Drehkopf versehen sein, sodaß auch von dieser Seite aus Fluide eingespeist werden können.

Die Kopplung kann alternativ auch dadurch erfolgen, daß die Antriebswelle 62 axial verschiebbar gelagert ist, um die Kupplung ein- und ausrücken zu können. In diesem Fall ist auf der entgegengesetzten Seite der Trommelwelle vorzugsweise ein Gegen-Drehlager vorgesehen, das die für die Abdichtung der Kupplungsanordnung 60 erforderliche Axialkraft aufnimmt. Die Antriebswelle kann auch eine Gleitdichtung aufweisen oder durch einen Tombakbalgen oder dergleichen abgedichtet sein.

Bei einer Abwandlung des oben beschriebenen Ausführungsbeispieles ist das U-förmige Düsenrohr 44 nicht an der Hohlwelle 40 sondern an dem sich in das Innere der Autoklavenkammer erstreckenden Teil der Antriebswelle 62 befestigt. Dies hat den Vorteil, daß das Düsenrohr 44 auch dann für Reinigungs- und Behandlungszwecke zur Verfügung steht, wenn eine-andere Reinigungsvorrichtung als die beschriebene Trommelreinigungsvorrichtung 36 verwendet wird. Man kann dann z.B. auch Gegenstände, die auf einer Palette oder in Wannen in die Autoklavenkammer eingebracht worden sind, mittels der nach innen gerichteten Sprühdüsen des Düsenrohres 44 reinigen, wobei man dann selbstverständlich den Drehbereich des Dünsenrohres 44 entsprechend beschränkt, z.B. auf die oberen 180°.

Gemäß einer weiteren Ausgestaltung der vorliegenden Reinigungsvorrichtung ist die Autoklavenkammer mit einer nicht dargestellten Vorrichtung zum Einspeisen von Mikrowellen versehen, wobei man sich ähnlicher Anordnungen bedienen kann, wie bei den bekannten Mikrowellenherden.

Zum Entladen braucht die Trommel-Reinigungsvorrichtung 36 nicht aus der Autoklavenkanmer entnommen zu werden, es genügt vielmehr, wie Figur 1 zeigt, auf der sterilen Seite eine Entladevorrichtung 92 mit einer Rutsche 94 und einem Aufnahmebehälter 96 an die Trommel anzusetzen sowie, gegebenenfalls mehrmals, die Trommel in Uhrzeigerrichtung anzutreiben, also in der entgegengesetzten Richtung wie beim Reinigen (Figur 2) und die Trommeltüre 40 zu öffnen.

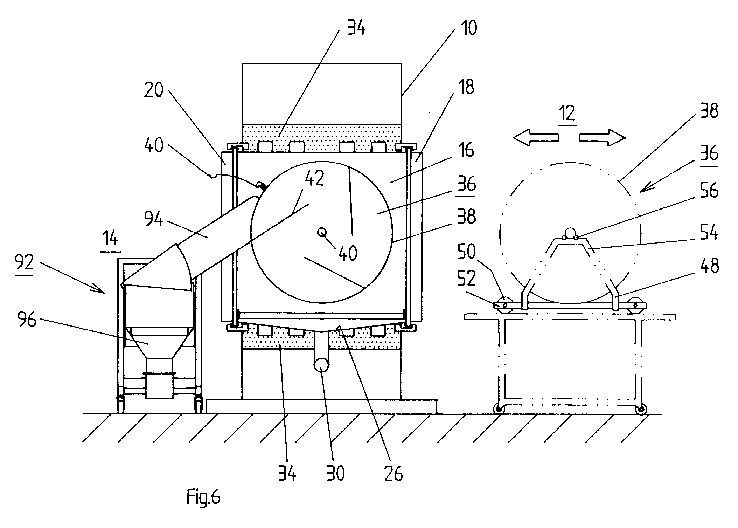

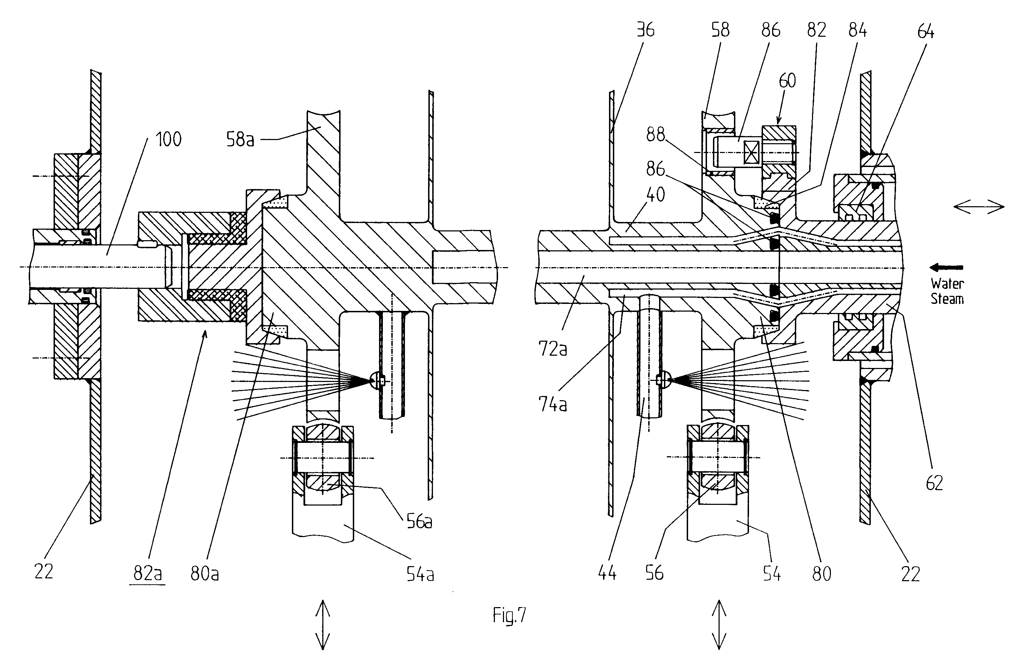

Die in den Figuren 6 und 7 dargestellte bevorzugte Ausführungsform der Erfindung entspricht zum großen Teil der Ausführungsform gemäß Figur 1 bis 5 und es wurden daher für entsprechende Teile die gleichen Bezugszeichen verwendet. Die Einrichtung gemäß Figur 6 und 7 unterscheidet sich in vorteilhafter Weise von der gemäß Figur 1 bis 5 darin, daß der Rahmen 48, auf dem die Trommel 38 beim Einführen in die Autoklavenkammer 16 gelagert ist, von der Trommel gelöst und unabhängig von letzterer aus der Autoklavenkammer entfernt werden kann. Der Rahmen 48 befindet sich also während des Reinigungsvorganges außerhalb der Autoklavenkammer und kann daher auch keine Quelle für Verunreinigungen in der Autoklavenkammer darstellen.

Die Abkopplung des Rahmens von der Trommel, nachdem die Trommelwelle mit dem Antrieb und dem Gegenlager gekoppelt worden ist, kann auf verschiedene Weise geschehen, wie anhand von Figur 7 erläutert werden soll.

Figur 7 zeigt die Enden der Trommelwelle im Axialschnitt. Das in Figur 7 rechte, antriebsseitige Ende ist im wesentlichen so ausgebildet wie bei Figur 4 mit der Ausnahme, daß die Hohlwelle 62 in an sich bekannter Weise durch einen nicht dargestellten Pneumatikzylinder oder dergleichen axial verschiebbar ist. Das in Figur 7 linke Ende der Trommelwelle weist wie das rechte Ende einen kegelstumpfförmigen Kupplungsteil 80a auf, der mit einem komplementären Kupplungsteil 82a zusammenwirkt, welche mit der axial verschiebbaren Welle 100 fest verbunden ist. Die Trommelwelle ist auf ihrem linken Ende außerdem mit einer weiteren Lagerscheibe 58a versehen, die auf Rollen 56a eines Bügels 54a läuft, der sich auf der anderen Seite des Rahmens wie der Bügel 54 befindet.

Die Kupplungsteile 82, 82a sind während des Einführens der Trommel in die Autoklavenkammer und bei deren Entnehmen aus der Autoklavenkammer zurückgezogen, so daß die Kupplungsteile 80 bzw. 80a frei sind und der Mitnehmerstift 86 ausgerückt ist. Nachdem die Trommel mittels des Gestelles oder Rahmens 48 in die Autoklavenkammer eingefahren worden ist, werden die Kupplungsteile 80, 80a nach innen gedrückt, sie greifen dann in die Kupplungsteile 82, 82a ein, die etwas tiefer liegen, so daß die Trommelwelle beim weiteren Andrücken der Kupplungsteile angehoben wird. Alternativ oder zusätzlich können die Rollen 56, 56a absenkbar ausgebildet sein, z.B. indem die Bügel 54 in der Nähe der Rollen 56, 56a ein Gelenk (nicht dargestellt) aufweisen und der Winkel zwischen den Schenkeln der Bügel 54 durch eine Spindel oder eine wirkungsgleiche Vorrichtung (nicht dargestellt) veränderbar ist, so daß die Rollen beim Spreizen der-Bügel abgesenkt und beim Zusammenziehen der Bügel wieder angehoben werden, wie in Figur 7 durch Doppelpfeile unterhalb der nur teilweise dargestellten Bügel 54, 54a dargestellt ist.

Die beschriebenen Ausführungsbeispiele lassen sich in der verschiedensten Weise abwandeln.

Das Düsenrohr 44 kann sowohl nach innen gerichtete als auch nach außen gerichtete Düsen aufweisen, wie es in Figur 5 dargestellt ist, was besonders dann zweckmäßig ist, wenn das Düsenrohr 44 mit der Antriebswelle 62 verbunden ist und sowohl zur Reinigung der Innenwand der Autoklavenkammer als auch zur Reinigung von Gegenständen in der Autoklavenkammer dienen soll.

Die Autoklavenkammer kann trommel- oder zylinderförmig sein, d. h. an die Stelle der durch die Seitenwände, den Boden und die obere Wand gebildete Umfangswand tritt eine zylindrische oder rohrförmige Umfangswand.

Die Reinigungstrommel kann auch für die Aufnahme von Kassetten konstruiert sein oder durch ein hierfür geeignetes Gestell ersetzt werden.

Anstelle des Mitnehmerstifes 86 können die Kupplungsteile 80, 82 mit einer Zahnung versehen sein, durch die eine formschlüssige Kopplung gewährleistet ist.

## Patentansprüche

1. Einrichtung zum Reinigen und Sterilisieren von Gegenständen, wie pharmazeutischen Verschlußelementen, mit
- einer Autoklavenkammer (16), welche eine erste Stirnseite mit einer durch eine erste Verschlußvorrichtung (18) dicht verschließbaren Beschickungsöffnung, eine dieser gegenüberliegende zweite Stirnseite mit einer durch eine zweite Verschlußvorrichtung (20) dicht verschließbaren Entladeöffnung und eine Umfangswand (22, 24, 26, 28) aufweist,
- einer sich in der Autoklavenkammer befindenden Reinigungsvorrichtung (36), welche eine zur Aufnahme der zu reinigenden Gegenstände dienende Trommel (38) mit durchbrochenem Mantel sowie eine Welle (40) zur drehbaren Lagerung der Trommel aufweist,
- einer zum Antrieb der Trommel dienenden, außerhalb der Autoklavenkammer angeordneten Antriebsvorrichtung (62, 68), die durch eine in einer ersten Seite der Autoklavenkammer (16) vorgesehene Durchbrechung mit der Trommel koppelbar ist, und
- einer Vorrichtung zum Einspeisen mindestens eines Fluids, wie einer Reinigungsflüssigkeit, in die Trommel,
**dadurch gekennzeichnet,** daß die Trommel (38) einen Rahmen (48) aufweist, mit dem sie in die Autoklavenkammer (16) einführbar ist, daß die Antriebsvorrichtung mindestens eine von der als Hohlwelle ausgebildeten Trommelwelle (40) getrennte Antriebs-Hohlwelle (62) aufweist, die mit einem Ende dicht und drehbar durch die erste Seite der Autoklavenkammer (16) in diese eingeführt ist und außerhalb der Autoklavenkammer mit einem Antriebsaggregat gekoppelt ist, und daß die Antriebswelle (62) und die Trommelwelle (40) einander gegenüberliegende, komplementäre Kupplungsteile (80, 82) zur mechanischen Kopplung und zur Verbindung von in den Wellen (40, 62) verlaufenden Fluidkanälen (72, 74; 72a, 74a) aufweisen.

2. Einrichtung nach Anspruch 1, **dadurch gekennzeichnet,** daß das der Antriebswelle (62) abgewandte Ende der Trommelwelle (40) einen zweiten Kupplungsteil aufweist, der mit einem komplementären Kupplungsteil (82a) kuppelbar ist, welcher in einer der ersten Seite entgegengesetzten zweiten Seite der Autoklavenkammer (16) gelagert ist.

3. Einrichtung nach Anspruch 1 oder 2, **dadurch gekennzeichnet,** daß die komplementären Kupplungsteile (80, 82; 80a, 82a) im wesentlichen kegelstumpfförmige Kupplungsflächen aufweisen und durch axiale Verschiebung in Bezug aufeinander koppelbar und entkoppelbar sind.

4. Einrichtung nach Anspruch 1, 2 oder 3, **dadurch gekennzeichnet,** daß der Rahmen (48) bei angekoppelter Trommelwelle unabhängig von der Trommel aus der Autoklavenkammer entfernbar ist.

5. Einrichtung nach Anspruch 4, **dadurch gekennzeichnet,** daß der Rahmen (48) mit der Trommel (38) durch die Beschickungsöffnung in die Autoklavenkammer (16) einführbar und ohne Trommel durch die Beschickungsöffnung aus der Autoklavenkammer entfernbar ist.

6. Einrichtung nach einem der Ansprüche 1 bis 5, **dadurch gekennzeichnet,** daß die Trommelwelle (40) mindestens an dem der Antriebswelle (62) zugewandten Ende eine Lagerscheibe (58) aufweist, die auf am Rahmen gelagerten Rollen (56) läuft und bei angekoppelter Trommelwelle von den Rollen abkoppelbar ist.

7. Einrichtung nach einem der vorhergehenden Ansprüche, **dadurch gekennzeichnet,** daß die Trommelwelle (40) radial nach außen gerichtete Düsen aufweist und daß vom Trommelmantel axial verlaufende Mitnehmerbleche (42) nach innen in Richtungen vorspringen, welche einen vorgegebenen Winkel mit der Radialrichtung bilden.

8. Einrichtung nach einem der vorhergehenden Ansprüche, **dadurch gekennzeichnet,** daß ein Düsenrohr (44) an dem sich in die Autoklavenkammer (16) erstreckenden Teil der Antriebswelle angebracht ist (Figur 5).

9. Einrichtung nach Anspruch 8, **dadurch gekennzeichnet,** daß das Düsenrohr (44) radial nach außen gerichtete und/oder radial nach innen gerichtete Düsen aufweist.

10. Einrichtung nach einem der vorhergehenden Ansprüche, **dadurch gekennzeichnet,** daß die Trommelwelle (40) und die Antriebswelle (62) jeweils mindestens zwei Fluidkanäle (72, 74, 72a, 74a) enthalten und daß die Antriebswelle an ihrem äußeren Ende mit einer Vorrichtung (76, 78) zum Einführen von Flüssigkeit und/oder Dampf und/oder Gas in die Fluidkanäle versehen ist.

11. Einrichtung nach einem der vorhergehenden Ansprüche, **dadurch gekennzeichnet,** daß die Autoklavenkammer mit einer Vorrichtung zum Einspeisen von Mikrowellen versehen ist.

12. Einrichtung nach einem der vorhergehenden Ansprüche, **dadurch gekennzeichnet,** daß die Innenwand der Autoklavenkammer mit Düsen (46) versehen ist, welche in das Innere der Autoklavenkammer gerichtet sind.

13. Einrichtung nach einem der vorhergehenden Ansprüche, **dadurch gekennzeichnet,** daß an der der Antriebswelle (62) gegenüberliegenden Seite der Autoklavenkammer (16) eine Widerlagerwelle vorgesehen ist und daß eine Vorrichtung (98) zum axialen Andrücken dieser Welle an die Trommelwelle (40) vorgesehen ist.

14. Einrichtung nach Anspruch 13, **dadurch gekennzeichnet,** daß die Widerlager-Welle eine Hohlwelle (100) und mit einer Vorrichtung zum Einspeisen von Fluid versehen und/oder mit einer Antriebsvorrichtung verbunden ist.

## Claims

1. Device for cleaning and sterilising articles, such as pharmaceutical closure elements, including
- an autoclave chamber (16) which has a first end face with a loading opening which may be sealingly closed by a first closure device (18), a second end face opposed to it with an unloading opening which may be sealingly closed by a second closure device (20) and a peripheral wall (22, 24, 26, 28),
- a cleaning device (36) which is located in the autoclave chamber and which has a drum (38) with a perforated wall for receiving the articles to be cleaned and a shaft (40) for rotatably mounting the drum,
- a drive device (62, 68) for driving the drum disposed outside the autoclave chamber, which device may be coupled to the drum through an opening provided in a first side of the autoclave chamber (16), and
- a device for feeding at least one fluid, such as a cleaning liquid, into the drum,
characterised in that the drum (38) comprises a frame (48) with which it may be introduced into the autoclave chamber (16), and the drive device has at least one hollow drive shaft (62) which is separate from the drum shaft (40) constructed as a hollow shaft and one end of which passes into the autoclave chamber (16) through the first side thereof in a manner such that it is sealed and rotatable and is coupled to a drive unit outside the autoclave chamber and that the drive shaft (62) and the drum shaft (40) have opposing complementary coupling portions (80, 82) for mechanically coupling and for connecting fluid passages (72, 74; 72a, 74a) extending in the shafts (40, 62).

2. Device as claimed in Claim 1, characterised in that the end of the drum shaft (40) remote from the drive shaft (62) has a second coupling portion which may be coupled to a complementary coupling portion (82a) which is mounted in a second side of the autoclave chamber (16) opposed to the first side.

3. Device as claimed in Claim 1 or 2, characterised in that the complementary coupling portions (80, 82; 80a, 82a) have substantially frustoconical coupling surfaces and may be coupled and decoupled by axial displacement with respect to one another.

4. Device as claimed in Claim 1, 2 or 3, characterised in that the frame (48) may be removed from the autoclave chamber when the drive shaft is coupled independently from the drum.

5. Device as claimed in Claim 4, characterised in that the frame (48) may be introduced with the drum (38) into the autoclave chamber (16) through the loading opening and may be removed from the autoclave chamber without the drum through the loading opening.

6. Device as claimed in one of Claims 1 to 5, characterised in that the drum shaft (40) has a bearing disc on at least that end directed towards the drive shaft (62), which disc runs on rollers (56) mounted on the frame and may be uncoupled from the rollers when the drum shaft is coupled.

7. Device as claimed in one of the preceding claims, characterised in that the drum shaft (40) has nozzles directed radially outwardly and that engagement plates (42) extending axially from the drum wall project inwardly in directions which define a predetermined angle with the radial direction.

8. Device as claimed in one of the preceding claims, characterised in that a nozzle tube (4) is connected to that portion of the drive shaft which extends into the autoclave chamber (16) (Figure 5).

9. Device as claimed in Claim 8, characterised in that the nozzle tube (44) has radially outwardly directed and/or radially inwardly directed nozzles.

10. Device as claimed in one of the preceding claims, characterised in that the drum shaft (40) and the drive shaft (62) each contain at least two fluid passages (72, 74, 72a, 74a) and that the drive shaft is provided at its outer end with an apparatus (76, 78) for introducing liquid and/or steam and/or gas into the fluid passages.

11. Device as claimed in one of the preceding claims, characterised in that the autoclave chamber is provided with an apparatus for introducing microwaves.

12. Device as claimed in one of the preceding claims, characterised in that the inner wall of the autoclave chamber is provided with nozzles (46) which are directed into the interior of the autoclave chamber.

13. Device as claimed in one of the preceding claims, characterised in that provided on the side of the autoclave chamber (16) opposite to the drive shaft (62) there is a support shaft and that an apparatus (98) is provided for axially pressing this shaft against the drum shaft (40).

14. Device as claimed in Claim 13, characterised in that the support shaft is a hollow shaft (100) and is provided with an apparatus for introducing fluid and/or is connected to a drive apparatus.

## Revendications

1. Dispositif pour le nettoyage et la stérilisation d'objets, comme des éléments pharmaceutiques de fermeture, comprenant
- une chambre d'autoclave (16), qui présente une première face frontale comportant une ouverture de chargement pouvant être fermée de façon étanche par un premier dispositif de fermeture (18), une deuxième face frontale opposée, comportant une ouverture de déchargement, pouvant être fermée de façon étanche par un deuxième dispositif de fermeture (20) et une paroi périphérique (22, 24, 26, 28),
- un dispositif de nettoyage (36), placé dans la chambre d'autoclave, qui présente un tambour (38) servant à recevoir les objets à nettoyer et comportant une enveloppe perforée ainsi qu'un arbre (40) pour le montage tournant du tambour,
- un dispositif d'entraînement (62, 68), servant à entraîner le tambour et disposé à l'extérieur de la chambre d'autoclave, qui peut être couplé avec le tambour au travers d'un perçage prévu dans une première face de la chambre d'autoclave (16), et
- un dispositif pour alimenter le tambour avec au moins un fluide tel qu'un fluide de nettoyage,
caractérisé en ce que le tambour (38) présente un cadre (48) avec lequel il peut être introduit dans la chambre d'autoclave (16), en ce que le dispositif d'entraînement présente au moins un arbre creux d'entraînement (62) séparé de l'arbre du tambour (40) réalisé sous la forme d'un arbre creux, cet arbre creux d'entraînement (62) pénétrant, avec une de ses extrémités, de façon étanche et avec possibilité de tourner, dans la chambre d'autoclave (16) au travers de la première face de celle-ci et étant accouplé, en dehors de la chambre d'autoclave, à une unité d'entraînement, et en ce que l'arbre d'entraînement (62) et l'arbre du tambour (40) présentent des pièces d'accouplement (80, 82) complémentaires et placées l'une en face de l'autre, servant à l'accouplement mécanique et au raccordement de canaux (72, 74; 72a, 74a) pour les fluides passant dans les arbres (40, 62).

2. Dispositif suivant la revendication 1, caractérisé en ce que l'extrémité de l'arbre du tambour (40), qui est située à l'opposé de l'arbre d'entraînement (62), présente une deuxième pièce d'accouplement qui peut s'accoupler avec une pièce d'accouplement complémentaire (82a), elle-même montée sur une deuxième face de la chambre d'autoclave (16), cette face étant située à l'opposé de la première face.

3. Dispositif suivant la revendication 1 ou la revendication 2, caractérisé en ce que les pièces d'accouplement complémentaires (80, 82; 80a, 82a) présentent essentiellement des surfaces d'accouplement en forme de troncs de cônes et peuvent être accouplées ou découplées en les faisant coulisser axialement l'une par rapport à l'autre.

4. Dispositif suivant la revendication 1, la revendication 2 ou la revendication 3, caractérisé en ce que le cadre (48) peut être enlevé de la chambre d'autoclave indépendamment du tambour, quand l'arbre du tambour est accouplé.

5. Dispositif suivant la revendication 4, caractérisé en ce que le cadre (48) avec le tambour (38) peut être introduit dans la chambre d'autoclave (16) au travers de l'ouverture de chargement, et être enlevé, sans le tambour, de la chambre d'autoclave au travers de l'ouverture de chargement.

6. Dispositif suivant l'une quelconque des revendications 1 à 5, caractérisé en ce que l'arbre du tambour (40) présente, au moins à son extrémité tournée vers l'arbre d'entraînement (62), un disque formant palier (58), qui tourne sur des galets (56) montés sur le cadre et qui est découplable des galets, quand l'arbre d'entraînement (62) est accouplé.

7. Dispositif suivant l'une quelconque des revendications précédentes, caractérisé en ce que l'arbre du tambour (40) présente des buses dirigées radialement vers l'extérieur et en ce que des tôles d'entraînement (42), partant axialement de l'enveloppe du tambour, font saillies vers l'intérieur, dans des directions qui forment un angle prédéterminé avec la direction radiale.

8. Dispositif suivant l'une quelconque des revendications précédentes, caractérisé en ce qu'un tube porte-buses (44) est monté sur la partie de l'arbre d'entraînement dirigée vers la chambre d'autoclave (16).

9. Dispositif suivant la revendication 8, caractérisé en ce que le tube porte-buses (44) présente des buses dirigées radialement vers l'extérieur et/ou des buses dirigées radialement vers l'intérieur.

10. Dispositif suivant l'une quelconque des revendications précédentes, caractérisé en ce que l'arbre du tambour (40) et l'arbre d'entraînement (62) contiennent chacun au moins deux canaux (72, 74; 72a, 74a) pour les fluides et en ce que l'arbre d'entraînement est muni, à son extrémité extérieure, d'un dispositif (76, 78) destiné à introduire du liquide et/ou de la vapeur et/ou du gaz dans les canaux pour fluides,

11. Dispositif suivant l'une quelconque des revendications précédentes, caractérisé en ce que la chambre. d'autoclave est équipée d'un dispositif pour l'alimentation de micro-ondes.

12. Dispositif suivant l'une quelconque des revendications précédentes, caractérisé en ce que la paroi intérieure de la chambre d'autoclave est équipée de buses (46) dirigées vers l'intérieur de la chambre d'autoclave.

13. Dispositif suivant l'une quelconque des revendications précédentes, caractérisé en ce que, sur la face de la chambre d'autoclave (16) située du côté opposé à l'arbre d'entraînement (62), il est prévu un arbre formant contre-appui et en ce qu'il est prévu un dispositif (98) destiné à pousser axialement cet arbre vers l'arbre (40) du tambour.

14. Dispositif suivant la revendication 13, caractérisé en ce que l'arbre formant contre-appui est un arbre creux (100) et est équipé d'un dispositif d'alimentation en fluide et/ou d'un dispositif d'entraînement.
